# EUROPÄISCHE PATENTSCHRIFT

(11) **EP 1 386 615 B1**
(45) Veröffentlichungstag und Bekanntmachung des Hinweises auf die Patenterteilung: **18.02.2009**
(21) Anmeldenummer: 03090092.2
(22) Anmeldetag: 04.04.2003
(51) Int. Cl.: A61K 38/18, A61K 39/395, C12N 15/18, A61K 31/7088, A61P 15/00, C12N 15/09, C12N 15/12, G01N 33/68, C07K 14/475

(54) **EG-VEGF/Prokineticin 2 Rezeptor Antagonisten**
EG-VEGF/Prokineticin 2 receptor antagonists
Antagonistes du récepteur EG-VEGF/prokineticin 2

(30) Priorität: 26.06.2002 DE 10229379
(43) Veröffentlichungstag der Anmeldung: 04.02.2004
(73) Patentinhaber: Bayer Schering Pharma Aktiengesellschaft, 13353 Berlin (DE)
(72) Erfinder: Haendler, Bernard, 13465 Berlin (DE); Hess-Stumpp, Holger, 13503 Berlin (DE); Schmidt, Anja, 10629 Berlin (DE)

(56) Entgegenhaltungen:
- WO-A-00/75327
- WO-A-02/00711
- WO-A-03/080892
- MASUDA Y ET AL: "ISOLATION AND IDENTIFICATION OF EG-VEGF/PROKINETICINS AS COGNATE LIGANDS FOR TWO ORPHAN G-PROTEIN-COUPLED RECEPTORS" BIOCHEMICAL AND BIOPHYSICAL RESEARCH COMMUNICATIONS, ACADEMIC PRESS, SAN DIEGO, CA, US, Bd. 293, Nr. 1, 26. April 2002 (2002-04-26), Seiten 396-402, XP008006065 ISSN: 0006-291X
- LIN DANIEL CHI-HONG ET AL: "Identification and molecular characterization of two closely related G protein-coupled receptors activated by prokineticins/endocrine gland vascular endothelial growth factor" JOURNAL OF BIOLOGICAL CHEMISTRY, Bd. 277, Nr. 22, 31. Mai 2002 (2002-05-31), Seiten 19276-19280, XP002262095 ISSN: 0021-9258
- LECOUTER JENNIFER ET AL: "Identification of an angiogenic mitogen selective for endocrine gland endothelium" NATURE, MACMILLAN JOURNALS LTD. LONDON, GB, Bd. 412, Nr. 6850, 30. August 2001 (2001-08-30), Seiten 877-884, XP002194811 ISSN: 0028-0836
- LECOUTER J ET AL: "The endocrine-gland-derived VEGF homologue Bv8 promotes angiogenesis in the testis: localization of Bv8 receptors to endothelial cells" PROCEEDINGS OF THE NATIONAL ACADEMY OF SCIENCES OF USA, NATIONAL ACADEMY OF SCIENCE. WASHINGTON, US, Bd. 100, Nr. 5, März 2003 (2003-03), Seiten 2685-2690, XP002964074 ISSN: 0027-8424
- WECHSELBERGER C ET AL: "The mammalian homologues of frog Bv8 are mainly expressed in spermatocytes" FEBS LETTERS, ELSEVIER SCIENCE PUBLISHERS, AMSTERDAM, NL, Bd. 462, Nr. 1-2, 26. November 1999 (1999-11-26), Seiten 177-181, XP004260611 ISSN: 0014-5793
- LI M ET AL: "IDENTIFICATION OF TWO PROKINETICIN CDNAS: RECOMBINANT PROTEINS POTENTLY CONTRACT GASTROINTESTINAL SMOOTH MUSCLE" MOLECULAR PHARMACOLOGY, BALTIMORE, MD, US, Bd. 59, Nr. 4, 2001, Seiten 692-698, XP001010437 ISSN: 0026-895X
- L. HULL ET AL: "Antiangiogenic agents are effective inhibitors of endometriosis" THE JOURNAL OF CLINICAL ENDOCRINOLOGY & METABOLISM, Bd. 88, Nr. 6, Seiten 2889-2899,

## Beschreibung

Die Erfindung betrifft EG-VEGF-/Prokineticin 2-Rezeptor Antagonisten und deren Verwendung als Arzneimittel für die Behandlung der Erkrankungen des Endometriums : Endometriose und dysfunktionelle Blutungen.

Endometriose ist eine der häufigsten gynäkologischen Erkrankungen, von der schätzungsweise 2-10% aller Frauen im reproduktionsfähigen Alter betroffen sind (Gazvani and Templeton 2002, Reproduction 123, 217-226; Smith 2001, Trends Endocrinol. Metab. 12, 147-151). Die Ursache ist die ektopische Implantation von endometrialem Gewebe überwiegend im Peritonealraum. Neben Schmerzen und zahlreichen anderen Symptomen sind viele Endometriosepatientinnen steril. Die Gründe für die Sterilität sind weitgehend unbekannt (Ryan and Taylor 1997, Obstet. Gynecol. Surv. 52, 365-371; Haney 1997, Reprod. Med. Rev. 6, 145-161).
Für die Behandlung der Endometriose ist derzeit keine Kausaltherapie verfügbar. Die Behandlung der Endometriose basiert auf dem Konzept des Östrogenentzugs. Nach einem invasiven Eingriff, bei dem während einer Laparoskopie oder Laparotomie Endometrioseherde entfernt oder verödet werden, schließt sich normalerweise eine medikamentöse Behandlung an, die durch die Induktion einer Hypoöstrogenität zu einer zumindest teilweisen Hemmung der Proliferation verbliebener, nicht zu erkennender Endometrioseherde führt (Sillem 1998, Programmed® 23, Suppl. 1, 1-28). Die verwendeten Medikamente haben zum Teil wegen ihrer Androgenität zahlreiche Nebenwirkungen, führen zu Durchbruchsblutungen, zu klimakterischen Beschwerden und zu Osteopenie. Obwohl sich die medikamentöse Behandlung für eine initiale Behandlung der Endometriose als effektiv erwiesen hat, konnte bislang keine Senkung der hohen Rezidivrate erreicht werden.

Der Endometriumkarzinom ist die vierthäufigste Krebsform in den westlichen Ländern. Abhängig vom Stadium der Diagnose beträgt die Überlebensrate nach 5 Jahren 27% bis 88%. Eine Hauptursache scheint ein erhöhter Östrogenspiegel zu sein (Akhmedkhanov et al. 2001, Ann. N. Y. Acad. Sci. 943, 296-315). Dieses führt zur vermehrten Proliferation von Zellen des Endometriums, was Fehler in der DNA-Replikation und somatische Mutationen verursachen kann und letztendlich zu einem malignen Tumor führen kann. Wie bei anderen Tumoren, spielt die Angiogenese eine essentielle Rolle im Krankheitsbild des endometrialen Karzinoms. Mehrere Studien haben eine erhöhte Vaskularisierung und eine Überexpression des endothelialen Wachstumsfaktors VEGF (vascular endothelial growth factor) und dessen Rezeptoren festgestellt (Abulafia et al. 1999, Gynecol. Oncol. 72, 220-231).
Zur Zeit werden mehrere Therapien verwendet, um das Endometriumkarzinom zu behandeln. Antiöstrogene zeigen gute Effekte, allerdings muss häufig auch eine Zusatztherapie mit Cytostatika bzw. Strahlen durchgeführt werden.

Circa 10% der Frauen leiden regelmäßig an starken Blutungen während der Menstruation verursacht durch Änderungen der Blutgefäße des Endometriums. Eine erhöhte Proliferationsrate der Endothelzellen wurde im Endometrium solcher Frauen nachgewiesen (Kooy 1996, Hum. Reprod. 11, 1067-1072). Zusätzlich wurden strukturelle Unterschiede in den Blutgefäßen beobachtet, wie zum Beispiel die unvollständige Ausbildung der glatten Muskelzellschicht (Abberton et al. 1999, Hum. Reprod. 14, 1072-1079). Da der Blutverlust während der Menstruation zum Teil durch die Konstriktion der Blutgefässe geregelt wird, liegt es nah, dass die Defekte der glatten Muskulatur wesentlich zur Blutung beitragen.
Neben dem operativen Eingriff (Hysterektomie) kann auch medikamentös behandelt werden. Auch hier kommen Steroidhormone zum Einsatz. Weiterhin werden nichtsteroidale anti-inflammatorische Substanzen verwendet, die sehr effektiv sind, jedoch viele Nebenwirkungen haben, so dass sie nur für relativ kurze Zeit benutzt werden können (Irvine and Cameron 2000, Baillieres Best Pract Res Clin Obstet Gynaecol. 13, 189-202).

Es besteht die dringende Notwendigkeit neue Behandlungsmethoden und Substanzen zu finden, die eine gezielte Behandlung der Erkrankungen des Endometriums ermöglichen. Die vorliegende Erfindung löst das Problem durch eine neue Therapiemethode, bei der EG-VEGF, Prokineticin 2 und die entsprechenden Rezeptoren als Zielsubstanz für neue Medikamente dienen.
EG-VEGF (endocrine gland derived vascular endothelial growth factor), auch als Prokineticin 1 bezeichnet und wurde bereits als spezifischer Wachstumsfaktor für Endothelzellen aus dem adrenergen Cortex der Nebenniere beschrieben. Seine Expression wurde im Ovar, der Testis, der Placenta und in der Nebenniere nachgewiesen (LeCouter et al. 2001, Nature 412, 877-884). Zusätzlich wurden kontraktile Effekte auf glatte Muskeln des Gastrointestinaltraktes nachgewiesen (Li et al. 2001, Mol. Pharmacol. 59, 692-698). In der WO 02/00711 (Genentech, Inc) wird die Nukleinsäuresequenz und die korrespondierende Aminosäuresequenz des EG-VEGF-Polypeptids offenbart, sowie deren Verwendung bei der Behandlung von Erkrankungen, die mit einem Hormon-produzierendem Gewebe (z.B. Ovar) in Zusammenhang stehen. EG-VEGF und Prokineticin 2 (auch Bv8 genannt) gehören derselben Familie an, sie weisen eine 60% Identität auf und sind zu 75 % gleichartig. Beide Proteine weisen einen konservierten Bereich auf, der 10 Cysteinreste umfasst (Wechselberger et al., 1999, FEBS Lett. 462, 177-181). Expression von Prokineticin 2 wurde fast ausschließlich im Hoden und im Gehirn nachgewiesen (Wechselberger et al., 1999, FEBS Lett. 462, 177-181; LeCouter et al., 2003, Proc. Natl. Acad. Sci. USA 100, 2685-2690). Prokineticin 2 fördert Angiogenese im Hoden (LeCouter et al., 2003, Proc. Natl. Acad. Sci. USA 100, 2685-2690), Kontraktion von glatten Muskeln des Gastrointestinaltraktes (Li et al. 2001, Mol. Pharmacol. 59, 692-698) und vermittelt den Tag/Nacht Rythmus des suprachiasmatischen Nucleus des Gehirns (Cheng et al. 2002, Nature 417, 405-410). Auf die Bedeutung von EG-VEGF oder Prokineticin 2 im Endometrium wird nicht eingegangen. Die Rolle von EG-VEGF oder Prokineticin 2 bei Erkrankungen des Endometriums war bisher nicht bekannt.

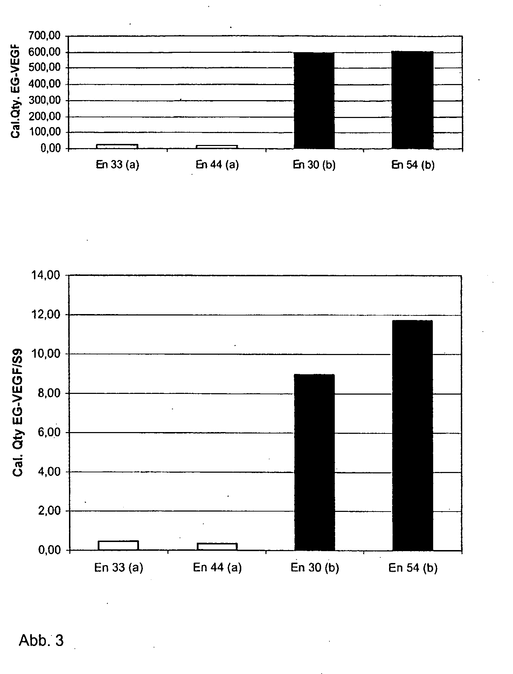

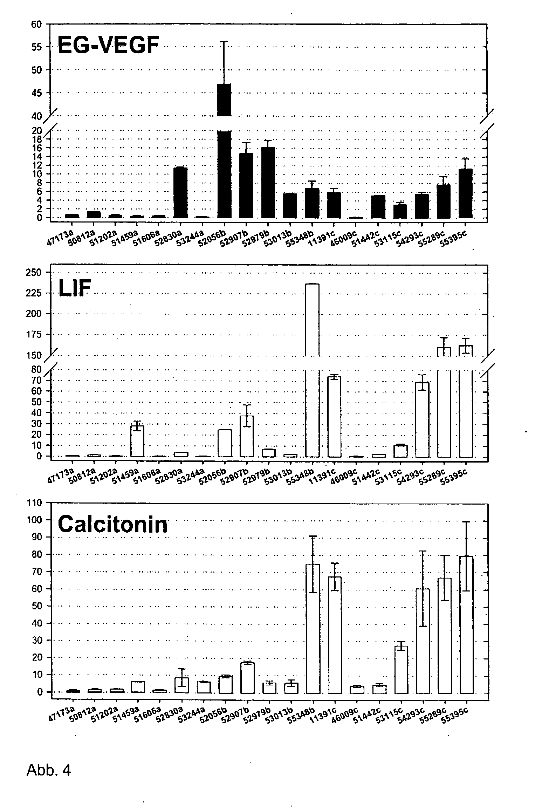

In der vorliegenden Erfindung konnte gezeigt werden, dass EG-VEGF im Endometrium exprimiert wird und im sogenannten lmplantationsfenster des Zyklus hochreguliert wird (Abb. 3 und 4). Mittels semi-quantitativer und quantitativer Bestimmungen wurde die mRNA aus dem humanen Endometrium dreier klinischer Studiengruppen miteinander verglichen. Das Ergebnis dieser Studien ergab, dass die mRNA von EG-VEGF im Vergleich zur Kontrollgruppe a (vor Öffnung des lmplantationsfensters) in der Gruppe b (Zeit des offenen lmplantationsfensters) bei gesunden Frauen hochreguliert ist. Auch in der Gruppe c (Endometriose-Patientinnen) wurde eine erhöhte Menge an EG-VEGF-mRNA festgestellt.
Die Bindung von EG-VEGF oder Prokineticin 2 an zwei verwandte G-Protein gekoppelte Rezeptoren (GPR73a und GPR73b) führt zur Aktivierung des Rezeptors.

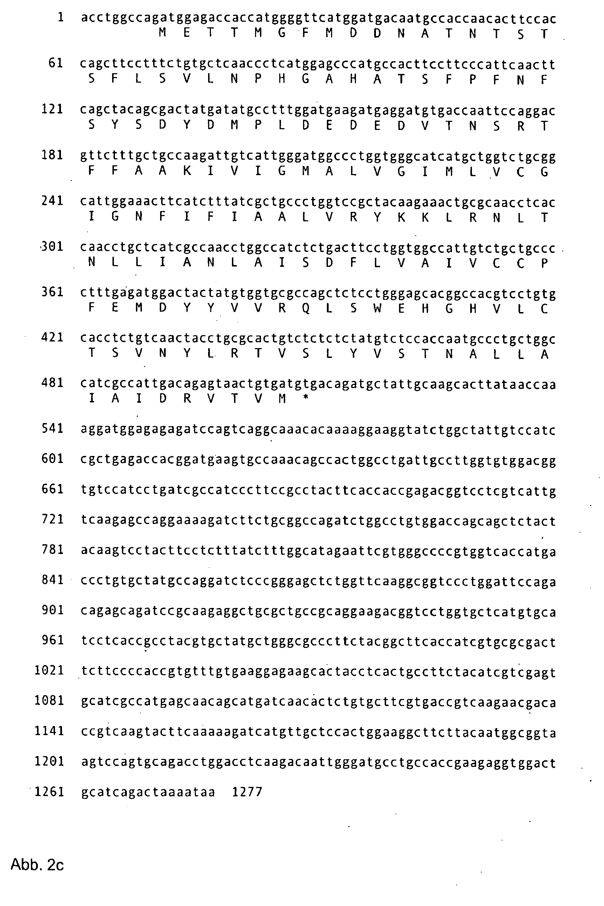

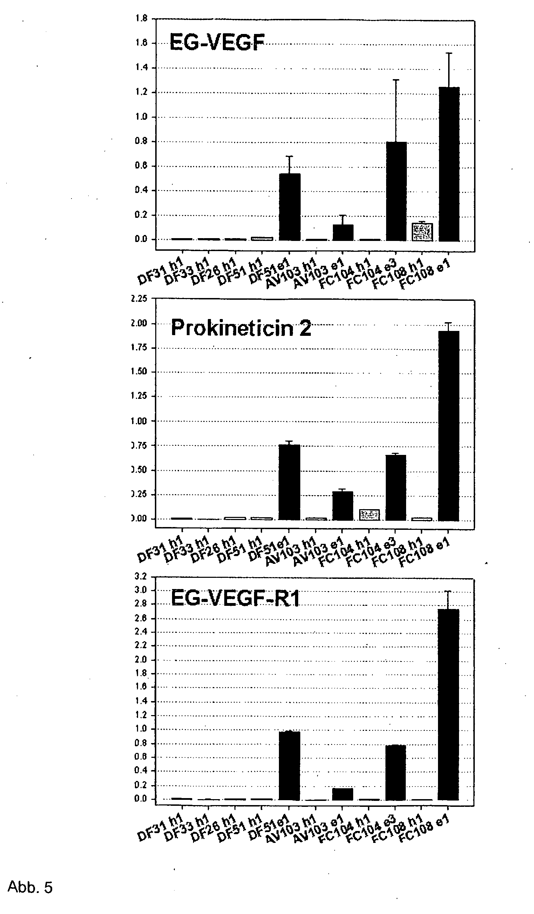

Diese physiologischen Bindungspartner des EG-VEGF- und des Prokineticin 2-Polypeptids wurden erst kürzlich identifiziert und charakterisiert (Lin et al. 2002, J. Biol. Chem. 277, 19276-19280). Die durch die Rezeptoren-vermittelten Effekte sind verantwortlich für die biologischen Effekte von EG-VEGF und Prokineticin 2, die wiederum für die pathologischen Erkrankungen des Endometriums verantwortlich sind.
In der vorliegenden Erfindung konnte gezeigt werden, dass die Expression des EG-VEGFs, des Prokineticin 2 und des EG-VEGF Rezeptors GPR73a in Läsionen, die aus verschiedenen, an Endometriose erkrankten Patientinnen stammen, deutlich höher ist als im gesunden Gewebe (Abb. 5).
Weiterhin konnte eine neue humane Splice-Variante von GPR73a identifiziert werden (Abb. 2c). Die RNA von dieser Variante enthält ein zusätzliches Exon, das allerdings ein Stop-Codon enthält und somit zu einem verkürzten Protein führt. Der verkürzte Rezeptor geht nur bis zur dritten Transmembrandomäne und sollte somit nicht in der Lage sein die Signaltransduktion zu vermitteln. Es könnte sich bei dem Rezeptor um einen dominant-negativen Inhibitor von GPR73a bzw. GPR73b handeln, der Liganden abfängt bzw. an Heterodimere bindet.
Eine Hemmung der Bindung des Wachstumsfaktors EG-VEGF oder Prokineticin 2 an die EG-VEGF-Rezeptoren einerseits bzw. eine direkte Hemmung des EG-VEGFoder Prokineticin 2 Polypeptids unterdrückt die biologische Funktion von EG-VEGF oder Prokineticin 2. Diese Hemmung ermöglicht die gezielte Behandlung der oben aufgeführten Erkrankungen. Die Erfindung stellt pharmazeutische Mittel für diese gezielte Behandlung bereit.

Die Erfindung liegt daher in der Verwendung einer pharmazeutischen Zusammensetzung, die als aktive Komponente eine hemmende Substanz, ausgewählt aus der Gruppe von:
einem Antikörper, der gegen EG-VEGF oder Prokineticin 2 gerichtet ist, einer EG-VEGF- oder Prokineticin 2-Antisense Nukleinsäure, einer EG-VEGF/Prokineticin 2-Rezeptor-Antisense-Nukleinsäure und einem Antikörper, der gegen einen EG-VEGF/Prokineticin 2-Rezeptor gerichtet ist, enthält, zur Herstellung eines Medikaments zur Behandlung oder Prävention von den Erkrankungen des Endometriums: Endometrioseund dysfunktionellen Blutungen.

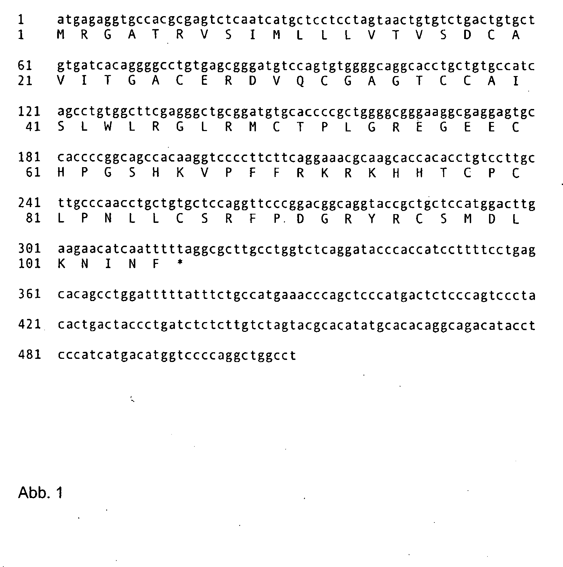

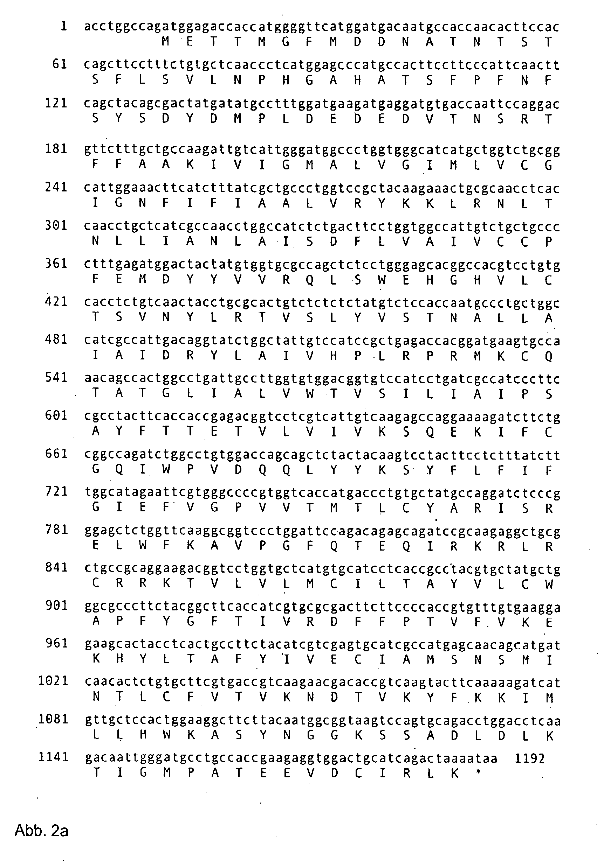

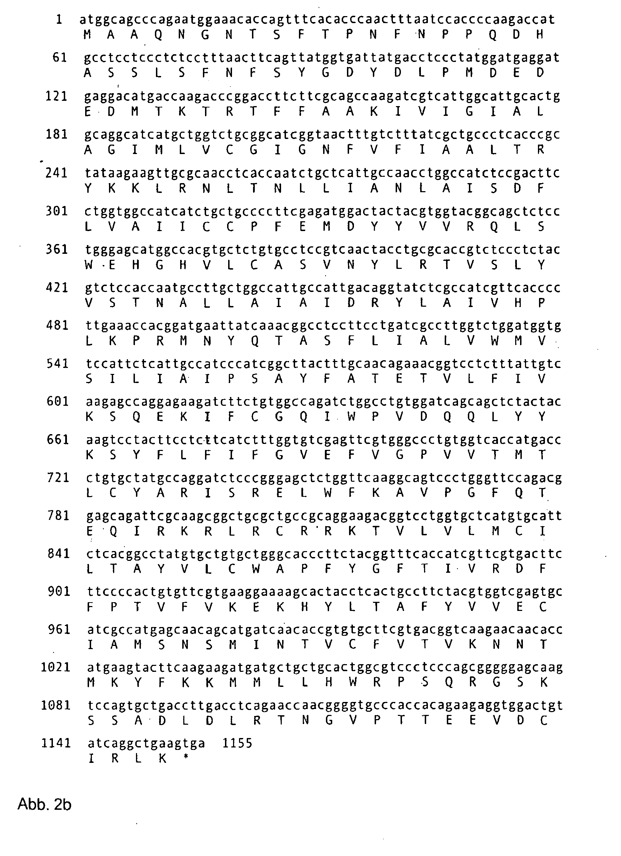

EG-VEGF-Nukleinsäure umfasst sowohl DNA, cDNA und RNA, bzw. Teile davon. Die DNA- und Proteinsequenzen sind in Abb. 1 dargestellt. Vorzugsweise ist die EG-VEGF-Nukleinsäure eine DNA.
Prokineticin 2-Nukleinsäure umfasst sowohl DNA, cDNA und RNA, bzw. Teile davon. Die DNA- und Proteinsequenzen sind in der GeneBank Datenbank unter Accession-Nummer NM_021935 beschrieben. Vorzugsweise ist die Prokineticin 2-Nukleinsäure eine DNA.
EG-VEGF/Prokineticin 2-Rezeptor-Nukleinsäure umfasst sowohl DNA, cDNA und RNA, bzw. Teile davon. Die DNA- und Proteinsequenzen sind in Abb. 2 a und 2b dargestellt. Die DNA- und Proteinsequenzen der neuen Splice-Variante sind in Abb. 2c dargestellt. Vorzugsweise ist die EG-VEGF/Prokineticin 2-Rezeptor-Nukleinsäure eine DNA.
Unter EG-VEGF- Polypeptid sind die gesamte Sequenz wie auch Teile davon zu verstehen.
Unter Prokineticin 2- Polypeptid sind die gesamte Sequenz wie auch Teile davon zu verstehen.
EG-VEGF-Antisense- Nukleinsäure ist eine DNA und / oder RNA, die komplementär zu einer EG-VEGF mRNA ist. Sie kann die gesamte komplementäre Sequenz oder Teilsequenzen umfassen.
Prokineticin 2-Antisense- Nukleinsäure ist eine DNA und / oder RNA, die komplementär zu einer Prokineticin 2 mRNA ist. Sie kann die gesamte komplementäre Sequenz oder Teilsequenzen umfassen.
EG-VEGF/Prokineticin 2-Rezeptor- Antisense- Nukleinsäure ist eine DNA und / oder RNA, die komplementär zu einer EG-VEGF/Prokineticin 2-Rezeptor mRNA ist. Sie kann die gesamte komplementäre Sequenz oder Teilsequenzen umfassen.
Ein Antikörper gegen EG-VEGF, Prokineticin 2 oder EG-VEGF/Prokineticin 2-Rezeptor kann monoklonal oder polyklonal sein. Er kann gegen EG-VEGF, Prokineticin 2 oder EG-VEGF/Prokineticin 2-Rezeptoren oder gegen Fragmente davon gerichtet sein. Die Gewinnung eines solchen Antikörpers erfolgt nach Standardmethoden durch Immunisierung von Versuchstieren.

Die pharmazeutischen Zusammensetzungen der Erfindung werden mit den üblichen festen oder flüssigen Trägerstoffen oder Verdünnungsmitteln und den üblichen pharmazeutischen und technischen Hilfsstoffen entsprechend der gewünschten Applikationsart mit einer geeigneten Dosierung in an sich bekannter Weise hergestellt. Tabletten können beispielsweise durch Mischen des Wirkstoffs mit bekannten Hilfsstoffen, beispielweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelatine, Gleitmittel wie Carboxypolymethylen, Carboxymethylcellulose, Celluloseacetatphthalat oder Polyvinylacetat, erhalten werden. Wirkstoff enthaltende Kapseln können beispielsweise hergestellt werden, indem man den Wirkstoff mit einem inerten Träger wie Milchzucker oder Sorbit mischt und in Gelatinekapseln einkapselt.
Die erfindungsgemäßen pharmazeutischen Zusammensetzungen können auch in geeigneten Lösungen wie beispielsweise physiologischer Kochsalzlösung zur Anwendung kommen.
Für die parenterale Applikation sind insbesondere ölige Lösungen, wie zum Beispiel Lösungen in Sesamöl, Rizinusöl und Baumwollsamenöl, geeignet. Zur Erhöhung der Löslichkeit können Lösungsvermittler, wie zum Beispiel Benzylbenzoat oder Benzylalkohol, zugesetzt werden.

Ebenfalls kann die erfindungsgemäße pharmazeutische Zusammensetzung zur Diagnose von Endometriose verwendet werden. Hierbei werden die Mengen an EG-VEGF oder Prokineticin 2 zum Beispiel durch einen ELISA-Test oder einen Protein-Chip bestimmt. Eine erhöhte Expression des EG-VEGFs oder des Prokineticin 2 zeigt das Vorliegen einer Endometriose an.

Die vorliegende Erfindung kann außerdem durch Verwendung der pharmazeutischen Zusammensetzung als Mittel zur Gentherapie Einsatz finden. Hierbei werden die Effekte von EG-VEGF oder Prokineticin 2 durch den Einsatz der Gentherapie blockiert. Dabei wird ein Vektor, der eine EG-VEGF- oder Prokineticin 2-Antisense Sequenz bzw. eine EG-VEGF/Prokineticin 2-Rezeptor-Antisense Sequenz enthält, konstruiert und appliziert. Beispiele sind Vektoren, die aus Adenovirus, Adenovirus-associated virus, Herpes simplex virus oder SV40 abgeleitet sind. Die Gentherapie kann wie beschrieben (Gomez-Navarro et al. 1999, Eur. J. Cancer, 35, 867-885) durchgeführt werden. Die Applikation kann lokal, d.h. direkt in den Uterus oder systemisch, d.h. über den Blutkreislauf erfolgen. Dies führt zu einer Blockade der Expression von EG-VEGF, von Prokineticin 2 bzw. des EG-VEGF/Prokineticin 2-Rezeptors im Endometrium. Dadurch wird die biologische Funktion des EG-VEGF oder des Prokineticin 2 gehemmt.

Ein weiterer Einsatz der Gentherapie besteht dadurch, dass ein Vektor, der die Sequenz der GPR73a-Splicevariante enthält, konstruiert und appliziert wird. Beispiele sind Vektoren, die aus Adenovirus, Adenovirus-associated virus, Herpes simplex virus oder SV40 abgeleitet sind. Die Gentherapie kann wie beschrieben (Gomez-Navarro et al. 1999, Eur. J. Cancer, 35, 867-885) durchgeführt werden. Die Applikation kann lokal, d.h. direkt in den Uterus oder systemisch, d.h. über den Blutkreislauf erfolgen. Dies führt zu einer Blockade der Funktion der EG-VEGF/Prokineticin 2-Rezeptoren GPR73a und/oder GPR73b im Endometrium. Dadurch wird die, durch EG-VEGF oder Prokineticin 2 induzierte, Signaltransduktion vorzeitig gestoppt.

Die vorliegende Erfindung betrifft ferner die Verwendung einer erfindungsgemäßen pharmazeutischen Zusammensetzung zum Nachweis uteriner Rezeptivität. Die uterine Rezeptivität ist eine Vorausetzung für eine erfolgreiche Einnistung der Blastozyste. Diese Einlagerung kann nur während einer kurzen Zeitspanne erfolgen, dem sogenannten Implantationsfenster, das beim Menschen während der mittleren sekretorischen Phase geöffnet ist, also etwa 7-9 Tage nach der Ovulation. Da die vorliegende Erfindung zeigt, dass zur Zeit des offenen Implantationsfensters die EG-VEGF Expression hochreguliert ist, kann durch Messung der Menge an EG-VEGF mRNA oder EG-VEGF-Polypeptid der geeignete Zeitpunkt für einen Transfer der mittels einer *in vitro* Fertilisierung befruchteten Eizelle ermittelt werden. Die Messung erfolgt mittels eines Northern Blots, einer PCR oder Chip-Hybridisierung oder durch einen Immuntest.

Weiterhin betrifft die Erfindung eine Methode zum Nachweis der uterinen Rezeptivität, wobei die Menge an EG-VEGF-Polypeptid und / oder die Menge an EG-VEGF-Nukleinsäure mit Hilfe einer erfindungsgemäßen pharmazeutischen Zusammensetzung bestimmt wird. Zunächst wird den Frauen eine Blut- oder Endometriumprobe entnommen und dann die Menge an EG-VEGF mRNA oder an EG-VEGF-Polypeptid bestimmt.

Beschrieben wird auch die Verwendung der pharmazeutischen Zusammensetzung zur Identifizierung von Substanzen, die die Effekte von EG-VEGF oder Prokineticin 2 beeinflussen. Effektoren sind Stoffe, die die biologische Aktivität des EG-VEGF- oder Prokineticin-2-Polypeptids beeinflussen, entweder durch Bindung an das EG-VEGF- oder Prokineticin 2-Polypeptid oder durch Kompetition mit dem EG-VEGF- oder Prokineticin 2-Polypeptid bei dessen Bindung an seinen physiologischen Bindungspartner. Effektoren können auch Substanzen sein, die bevorzugt an die Rezeptor Splice-Variante binden und somit die Funktion von GPR73a oder GPR73b modulieren bzw. hemmen. Effektoren können niedermolekulare Stoffe sein oder auch Peptide oder Proteine wie z.B. Antikörper. Eine weitere Möglichkeit stellen Nukleinsäure wie Spiegelmere oder Aptamere dar, die aufgrund ihrer räumlichen Struktur das Zielmolekül spezifisch binden können und damit eine Interaktion eines Liganden mit seinem Rezeptor verhindern können. Zur Identifizierung solcher Effektoren wird eine Zelle, die einen EG-VEGF-/Prokineticin 2-Rezeptor exprimiert, mit EG-VEGF oder Prokineticin 2 behandelt. Dies führt zu einer Veränderung des intrazellulären Calciumspiegels, was wiederum gemessen wird. Antagonisten sind Substanzen, die den Calcium-Einstrom hemmen können. Weiter können die Rezeptor Splice-Varianten zusammen mit GPR73a bzw. GPR73b coexprimiert werden, um nach Substanzen zu suchen, die selektiv die Heterodimerisierung hervorrufen und somit die EG-VEGF oder Prokineticin 2 induzierten Signale blockieren. Es können auch weitere Nachweissysteme für das Screening von EG-VEGF-/Prokineticin 2-Antagonisten verwendet werden.

### Beschreibung der Abbildungen

Abb. 1 zeigt die DNA- und Protein-Sequenz des humanen EG-VEGF.

Abb. 2 zeigt die DNA- und Protein-Sequenz der humanen EG-VEGF/Prokineticin 2-Rezeptoren GPR73a (2a), GPR73b (2b) und der neuen Splice-Variante von GPR73a (2c).

Abb. 3 zeigt die Expression der RNA von EG-VEGF im Endometrium nachgewiesen durch die RT-PCR-Methode. Eine Hochregulation des humanen EG-VEGF-Gens in der Gruppen b (LH+7-9/Fertil) im Vergleich zur Gruppe a (LH+2-4) ist eindeutig. Spezifische "Primer" für das EG-VEGF wurden benutzt. Als interne Kontrolle wurde eine Antisense-S9-Sonde amplifiziert. Nach der PCR-Amplifizierung wurden die Produkte auf einem Agarose-Gel getrennt und mit Ethidiumbromid gefärbt. Die Intensität der Banden wurde quantifiziert. Gruppe a: vor der Öffnung des lmplantationsfensters; Gruppe b: Zeit der geöffneten lmplantationsfensters bei gesunden Frauen; LH: luteinisierendes Hormon; 2-4 bzw. 7-9: Zeitraum in Tagen.

Abb. 4 zeigt die Expression der RNA von EG-VEGF im Endometrium nachgewiesen durch die TaqMan-Methode. Eine Hochregulation des humanen EG-VEGF-Gens in den Gruppen b (LH+7-9/Fertil.) und c (LH+7-9/Endometriose), im Vergleich zur Gruppe a (LH+2-4) ist eindeutig. Spezifische "Primer" für das EG-VEGF wurden benutzt. Zusätzlich wurden die Mengen and LIF und Calcitonin mRNA, zwei Gene die bekannter Weise zum Zeitpunkt des lmplantationsfensters hochreguliert werden, bestimmt. Die Mengen an PCR-Produkten wurde durch Färbung mit SyberGreen bestimmt. Alle mRNA Mengen wurden zur Aktin mRNA Menge abgeglichen. Gruppe a: vor der Öffnung des lmplantationsfensters; Gruppe b: Zeit der geöffneten Implantationsfensters bei gesunden Frauen; Gruppe c: Zeit des geöffneten lmplantationsfensters bei Endometriosepatientinnen; LH: luteinisierendes Hormon; 2-4 bzw. 7-9: Zeitraum in Tagen.

Abb. 5 zeigt die Expression der RNA des EG-VEGF und des EG-VEGF-Rezeptors GPR73a (hier EG-VEGF R1 genannt) in Läsionen, die aus an Endometriose erkrankten Patientinnen stammen, nachgewiesen durch die TaqMan-Methode. Die relative mRNA-Expression wurde zur Cyclophilin-mRNA Menge abgeglichen. Bei den Patientinnen # DF31h1, DF33h1 und DF26h1 handelt es sich um eutopes Gewebe von gesunden Kontrollpatientinnen (weiße Balken), die Proben der Endometriose-Patientinnen AV103, AV104, FC108 und DF51 stammen aus dem Endometrium (eutop, h1; graue Balken) oder aus ektopen Läsionen (e1 oder e3; schwarze Balken). Eine Hochregulation des humanen Gens für GPR73a (EG-VEGF R1) in verschiedenen Läsionen im Vergleich zu gesundem Gewebe ist eindeutig. Spezifische "Primer" für den GPR73a (EG-VEGF R1) wurden benutzt. Als Kontrolle wurden die GPR73a (EG-VEGF R1)-RNA-Mengen im Hoden und im Endometrium gemessen. Die Mengen an PCR-Produkten wurde durch Färbung mit SyberGreen bestimmt.

### Beispiele

### Beispiel 1: Bestimmung der EG-VEGF-RNA Mengen in endometrialen Proben durch semi-quantitative RT-PCR Analyse

1. Gruppe a (LH plus 2-4 Tage) : 2-4 Tage nach Auslösung der Ovulation durch die Ausschüttung des Luteinisierendes Hormons (LH) ist das Endometrium noch nicht rezeptiv für einen Embryo. Diese Gruppe setzte sich aus Patientinnen zusammen, die aufgrund eines Tubenverschlusses oder einer Sterilität des Mannes an einem IVF Programm teilnahmen und ein normales Endometrium aufwiesen.
2. Gruppe b (LH plus 7-9 Tage): Zu diesem Zeitpunkt ist das "lmplantationsfenster" offen und das Endometrium rezeptiv für die Einlagerung der Blastozyste. Durch das Öffnen des "Implantationsfensters" wird die Expression bzw. Hemmung von Genen erwartet, dessen Protein-Produkte eine essentielle Rolle in der Einnistung des Embryos spielen.
3. Gruppe c (LH plus 7-9 Tage plus Endometriose) : Zu diesem Zeitpunkt ist das lmplantationsfenster bei gesunden Frauen offen. Die Patientinnen dieser Gruppe waren aber aus unbekannten Gründen steril und litten an Endometriose.

Endometrium wurde von Patientinnen, die zu den zwei Gruppen LH+2-4/Fertil (a) und LH+7-9/Fertil (b) gehören, entnommen und in flüssigem Stickstoff schockgefroren. Daraus wurde Gesamt-RNA mit dem RNeasy Kit (Qiagen) isoliert. Anschließend wurde ein DNase l-Verdau (Invitrogen) gemacht. Beginnend mit 5,7 µg Gesamt-RNA wurde dann eine Erststrang-Synthese mit dem ProSTAR First Strand RT-PCR Kits von Stratagene durchgeführt. Für die semi-quantitative Bestimmung der EG-VEGF-Mengen wurden 0,5 µl Erststrang DNA und spezifische Primer für EG-VEGF (5'-TCAATCATGCTCCTCCTAGTAACTGTG-3' und 5'-AAGTCCATGGAGCAGCGGTAC-3') eingesetzt. Die PCR-Bedingungen waren: 2 Min. 94 °C; 20 Sek. 94 °C, 20 Sec. 65 °C, 40 Sek. 72 °C (40 Zyklen); 10 Min. 72 °C. Als interne Kontrolle wurde S9-RNA amplifiziert (Primer: 5'-AGGACCCACGGCGTCTGTTCG-3' und 5'-ATCCAGCAGCCCAGGAGGGAC-3'). Die PCR-Bedingungen waren: 2 Min. 94 °C; 20 Sek. 94 °C, 20 Sec. 60 °C, 40 Sek. 72 °C (24 Zyklen); 10 Min. 72 °C). Nach der Reaktion wurden die Amplifizierungsprodukte auf einem 2% Agarosegel getrennt und mit Ethidiumbromid gefärbt. Die Intensität der Banden wurde mit dem GelDoc-Gerät bestimmt. (Abb. 3)

### Beispiel 2: Bestimmung der EG-VEGF-RNA Mengen in endometrialen Proben durch Real-time quantitative RT-PCR Analyse

Endometrium wurde von Patientinnen, die den drei Gruppen LH+2-4/Fertil (a), LH+7-9/Fertil (b), LH+7-9/Endometriose (c) gehören, entnommen und in flüssigem Stickstoff schockgefroren. Aus den durch "Mörsern" unter flüssigem Stickstoff pulverisierten Geweben wurde mittels TRIZOL (Invitrogen) die Gesamt-RNA isoliert. Ausgehend von 5 µg Gesamt-RNA wurde zunächst ein DNase l-Verdau (Invitrogen) und anschließend eine Erststrang-Synthese unter Verwendung des SUPER-SCRIPT II First-Strand Synthesis System für RT-PCR (Invitrogen) durchgeführt. Für die Amplifizierung der Transkripte zur relativen Quantifizierung wurden 0,125 µl Erststrang-DNA eingesetzt. Unter Verwendung von spezifischen Primer Paaren (EG-VEGF: 5'-CTTCTTCAGGAAACGCAAGCA-3' und 5'-CGGGAACCTGGAGCACAG-3'; Calcitonin: 5'-CAACTTTGTGCCCACCAATGT-3' und 5'-GAGTCATTCAGCTGCTCAGGC-3'; Leukaemia inhibitory factor (LIF): 5'-CTAGTTCCCCACCTCAATCCC-3' und 5'-AAGGCCATGTGCTTTTCAGTG-3') und dem SYBR Green PCR Master Mix (PE Biosystems) wurde die Amplifizierung unter folgenden PCR-Bedingungen durchgeführt: 10 Min. 95 °C; 15 Sek. 95 °C, 1 Min. 60 °C (40 Zyklen). Als interne Kontrolle wurde die RNA für die Untereinheit 1A der Cytochromoxidase amplifiziert (Primer: 5'-CGTCACAGCCCATGCATTTG-3' und 5'-GGTTAGGTCTACGGAGGCTC-3'). Die Messung der Fluoreszenz als Maß für die Zunahme von Amplifizierungsprodukten erfolgte online mittels eines ABI Prism 7700 Sequence Detectors (PE Biosystems). Die Reinheit der Amplifizierungsprodukte wurde durch die Aufnahme von Schmelzkurven überprüft. (Abb. 4)

### Beispiel 3: Bestimmung der EG-VEGF, Prokineticin 2 und EG-VEGF-Rezeptor (GPR73a) RNA Mengen in Läsionen von Patientinnen.

Das Gewebe wurde nach Entnahme in flüssigem Stickstoff schockgefroren. Aus den durch "Mörsern" unter flüssigem Stickstoff pulverisierten Geweben wurde mittels TRIZOL (Invitrogen) die Gesamt-RNA isoliert. Eine Anreicherung der PolyA⁺ - RNA erfolgte im Batch-Verfahren unter Verwendung von Oligo-dT-Cellulose (Pharmacia). Ausgehend von 100 - 400 ng PolyA⁺ - RNA wurde eine Erststrang-Synthese unter Verwendung des SUPER-SCRIPT II First-Strand Synthesis System für RT-PCR (Invitrogen) durchgeführt. Für die Amplifizierung der Transkripte zur relativen Quantifizierung wurden 0,25 µl Erststrang-DNA eingesetzt. Unter Verwendung von spezifischen Primer Paaren (EG-VEGF: 5'-CTTCTTCAGGAAACGCAAGCA-3' und 5'-CGGGAACCTGGAGCACAG-3'; EG-VEGF Rezeptor GPR73a: 5'-ATTGACAGGTATCTGGCTATTGTCC-3' und 5'-CCAAGGCAATCAGGCCAGT-3'), dazugehörigen TaqMan-Sonden (EG-VEGF: 5'-TET-ACACCTGTCCTTGCTTGCCCAACC-TAMRA-3'; EG-VEGF Rezeptor GPR73a: 5'-FAM-CTGAGACCACGGATGAAGTGCCAAACA-TAMRA-3') und dem TaqMan PCR Master Mix (PE Biosystems) wurde die Amplifizierung als Multiplex-PCR unter folgenden PCR-Bedingungen durchgeführt: 2 Min. 50°C; 10 Min. 95 °C; 15 Sek. 95 °C, 1 Min. 62 °C (40 Zyklen). Als interne Kontrolle wurde die RNA für Cyclophilin amplifiziert (Primer: 5'-GAAGTTGGCCGCATGAAGA-3' und 5'-GCCTAAAGTTCTCGGCCGT-3'; Sonde: 5'-VIC-CGAGCTCTTTGCAGACGTTGTGCCT-TAMRA-3'). Die Messung der Fluoreszenz als Maß für die Zunahme von Amplifizierungsprodukten erfolgte online mittels eines ABI Prism 7700 Sequence Detectors (PE Biosystems).
Für die relative Quantifizierung der Prokineticin 2 - RNA wurde 0,5 µl Erststrang-DNA eingesetzt, ausgehend von PolyA⁺ - RNA. Unter Verwendung von spezifischen Primer Paaren (5'-CTCCTGTCATGGCACGGAA-3' und 5'-AGCCCAACAGCAGAGCTGAA-3') und dem SYBR Green PCR Master Mix (Eurogentec) wurde die Amplifizierung unter folgenden PCR-Bedingungen durchgeführt: 2 Min. 50°C; 10 Min. 95 °C; 15 Sek. 95 °C, 1 Min. 60 °C (40 Zyklen). Als interne Kontrolle wurde wiederum die RNA für Cyclophilin amplifiziert. (Abb. 5)

### Beispiel 4: Testsystem zum Auffinden von Rezeptor-Antagonisten des EG-VEGF-/Prokineticin 2-Rezeptors

Es wird eine Zelllinie (z. B. CHO oder HEK) etabliert, die die EG-VEGF-/Prokineticin 2-Rezeptoren GPR73a, GPR73b oder die Splice-Variante von GPR73a transient oder stabil exprimiert. Nach Zugabe eines Liganden wird eine Substanzdatenbank untersucht um Substanzen zu finden, die die Ligandenbindung verdrängen (Bindungsassay). Alternativ wird ein funktioneller Assay durchgeführt. Die durch den Liganden verursachte Calciumfreisetzung wird gemessen, zum Beispiel durch einen Aequorin-basierten Lumineszenz-Assay. Hier wird ein Expressionsplasmid für Aequorin in die Zellen mittransfiziert. Aequorin wird durch das freigesetzte Calcium gebunden, dies führt zur Erhöhung der Lumineszenz. Die Hemmung dieser Aktivität durch einen Rezeptor-Antagonisten wird als Messgröße für das Testen verwendet.

## Patentansprüche

1. Verwendung einer pharmazeutischen Zusammensetzung, die als aktive Komponente eine hemmende Substanz, ausgewählt aus der Gruppe von einem Antikörper der gegen EG-VEGF Polypeptid gerichtet ist, einer EG-VEGF-Antisense Nukleinsäure, einem Antikörper der gegen Prokineticin 2 gerichtet ist, einer Prokineticin 2-Antisense Nukleinsäure, einer EG-VEGF-/Prokineticin 2-Rezeptor-Antisense-Nukleinsäure und einem Antikörper, der gegen einen EG-VEGF-/Prokineticin 2-Rezeptor gerichtet ist, enthält, zur Herstellung eines Medikaments zum Nachweis der uterinen Rezeptivität oder zur Behandlung oder Prävention der folgenden Erkrankungen des Endometriums: Endometriose, dysfunktionelle Blutungen.

2. Verwendung einer pharmazeutischen Zusammensetzung, die als aktive Komponente eine hemmende Substanz, ausgewählt aus der Gruppe von einem Antikörper der gegen EG-VEGF Polypeptid gerichtet ist, einer EG-VEGF-Antisense Nukleinsäure, einem Antikörper der gegen Prokineticin 2 gerichtet ist, einer Prokineticin 2-Antisense Nukleinsäure, einer EG-VEGF-/Prokineticin 2-Rezeptor-Antisense-Nukleinsäure und einem Antikörper, der gegen einen EG-VEGF-/Prokineticin 2-Rezeptor gerichtet ist, enthält, zur Diagnostik von Endometriose.

3. Methode zum Nachweis der uterinen Rezeptivität in Endometrium-Proben von Patientinnen, **dadurch gekennzeichnet, dass** mittels eines Antikörpers, der gegen das EG-VEGF Polypeptid oder gegen Prokineticin 2 gerichtet ist und / oder durch Bestimmung der EG-VEGF- oder der Prokineticin 2 mRNA, die erhöhen Mengen an EG-VEGF-Polypeptid und /oder an EG-VEGF-Nukleinsäure bestimmt werden.

## Claims

1. Use of a pharmaceutical composition that as an active component comprises an inhibiting substance selected from the group consisting of: an antibody that is directed against EG-VEGF polypeptide, an EG-VEGF-antisense nucleic acid, an antibody that is directed against prokineticin 2, a prokineticin 2-antisense nucleic acid, an EG-VEGF/prokineticin 2-receptor-antisense nucleic acid and an antibody that is directed against EG-VEGF/prokineticin 2 receptor, in the manufacture of a medication for detecting uterine receptivity or for treating or preventing the following diseases of the endometrium: endometriosis, dysfunctional bleeding.

2. Use of a pharmaceutical composition that as an active component comprises an inhibiting substance that is selected from the group consisting of: an antibody that is directed against EG-VEGF polypeptide, an EG-VEGF-antisense nucleic acid, an antibody that is directed against prokineticin 2, a prokineticin 2-antisense nucleic acid, an EG-VEGF/prokineticin 2-receptor-antisense nucleic acid and an antibody that is directed against an EG-VEGF/prokineticin 2 receptor, for the diagnosis of endometriosis.

3. Method for detecting uterine receptivity in samples of endometrium of patients, **characterized in that** the increased amounts of EG-VEGF polypeptide and/or of EG-VEGF nucleic acid are determined with the aid of an antibody that is directed against EG-VEGF polypeptide or against prokineticin 2 and/or by determination of EG-VEGF or prokineticin 2 mRNA.

## Revendications

1. Utilisation d'une composition pharmaceutique qui en tant que composant actif contient une substance inhibitrice choisie dans le groupe consistant en un anticorps qui est dirigé contre le polypeptide EG-VEGF, un acide nucléique antisens de l'EG-VEGF, un anticorps qui est dirigé contre la prokinéticine 2, un acide nucléique antisens de la prokinéticine 2, un acide nucléique antisens du récepteur de l'EG-VEGF/prokinécitine 2, et un anticorps qui est dirigé contre un récepteur de l'EG-VEGF/prokinéticine 2, pour préparer un médicament destiné à la détection de la réceptivité utérine ou au traitement ou à la prévention des maladies suivantes de l'endomètre : endométriose, hémorragies dysfonctionnelles.

2. Utilisation d'une composition pharmaceutique qui en tant que composant actif contient une substance inhibitrice choisie dans le groupe consistant en un anticorps qui est dirigé contre le polypeptide EG-VEGF, un acide nucléique antisens de l'EG-VEGF, un anticorps qui est dirigé contre la prokinéticine 2, un acide nucléique antisens de la prokinéticine 2, un acide nucléique antisens du récepteur de l'EG-VEGF/prokinécitine 2, et un anticorps qui est dirigé contre un récepteur de l'EG-VEGF/prokinéticine 2, pour le diagnostic de l'endométriose.

3. Procédé de détection de la réceptivité utérine dans des échantillons d'endomètre de patientes, **caractérisé en ce qu'**on détermine, à l'aide d'un anticorps qui est dirigé contre le polypeptide EG-VEGF ou contre la prokinéticine 2, et/ou par détermination de l'ARNm de l'EG-VEGF ou de la prokinéticine 2, les quantités accrues du polypeptide EG-VEGF et/ou de l'acide nucléique de l'EG-VEGF.
